Europäisches Patentamt

European Patent Office

Office européen des brevets

(11) Publication number: **0 471 866 A1**

# EUROPEAN PATENT APPLICATION

(21) Application number: **90115940.0**

(22) Date of filing: **20.08.90**

(51) Int. Cl.⁵: **C08B 11/193**

(43) Date of publication of application:
**26.02.92 Bulletin 92/09**

(84) Designated Contracting States:
**CH DE FR GB LI**

(71) Applicant: **Shin-Etsu Chemical Co., Ltd.**
**6-1, Otemachi 2-chome**
**Chiyoda-ku Tokyo 100(JP)**

(72) Inventor: **Obara, Sakae, SHIN-ETSU CHEMICAL**
**Co., LTD.**
**Synthetic Tech. Lab., 28-1, Oaza**
**Nishi-Fukushima**
**Kubiki-mura, Nakakubiki-gun, Nigata 942(JP)**
Inventor: **Muto, Hiroaki, SHIN-ETSU CHEMICAL**
**Co., LTD.**
**Synthetic Tech. Lab., 28-1, Oaza**
**Nishi-Fukushima**
**Kubiki-mura, Nakakubiki-gun, Nigata 942(JP)**
Inventor: **Chiba, Tohru, SHIN-ETSU CHEMICAL**
**Co., LTD.**
**Synthetic Tech. Lab., 28-1, Oaza**
**Nishi-Fukushima**
**Kubiki-mura, Nakakubiki-gun, Nigata 942(JP)**
Inventor: **Tanioka, Soji, SHIN-ETSU CHEMICAL**
**Co., LTD.**
**6-1, Otemachi 2-chome, Chiyoda-ku**
**Tokyo 100(JP)**

(74) Representative: **Bühling, Gerhard, Dipl.-Chem.**
**et al**
**Patentanwaltsbüro Tiedtke-Bühling-Kinne**
**Grupe-Pellmann-Grams-Struif Bavariaring 4**
**W-8000 München 2(DE)**

(54) **Modified water-soluble cellulose ether.**

(57) A modified water-soluble cellulose ether shows a high viscosity-developing effect in only a small amount and is useful as a thickening agent for cement mortar, materials for extrusion molding, paints, cosmetic formulations, detergents or the like. The modified cellulose ether can be obtained according to a method comprising the step of reacting a water-soluble cellulose ether having a weight average molecular weight ranging from 10,000 to 3,000,000 and carrying alkyl groups having 1 to 3 carbon atoms and/or hydroxyalkyl groups having 2 to 4 carbon atoms with a glycidyl ether carrying an alkyl group having 12 to 26 carbon atoms to thus substitute the hydroxyl groups in the cellulose ether molecule in a ratio ranging from 0.001 to 0.1 mole per mole of the glucose ring constituting the water-soluble cellulose ether.

The present invention relates to a modified water-soluble cellulose ether which is useful as a thickening agent for cement mortar, materials for extrusion molding, paints, cosmetic formulations, detergents or the like as well as a method for preparing the same.

Water-soluble cellulose ethers have widely been used as thickening agents or an agent for water retention in a variety of fields and, in each field, those having optimum physical properties, i.e., those which satisfy the requirements for each application are properly selected and practically used. The physical properties thereof are exclusively influenced by mainly the kinds of the substituents and the degree of substitution of the water-soluble cellulose ether as well as the degree of polymerization of the cellulose chain.

When they are employed as thickening agents, it is very important to minimize the amount of the cellulose ether required for imparting a desired viscosity to a solution to which the cellulose ether is added. To this end, a cellulose ether having a high degree of polymerization must be used. However, it is very difficult to precisely control the degree of polymerization in the preparation of a cellulose ether having such a high degree of polymerization and hence cellulose ether products having uniform quality cannot easily be obtained. Under such circumstances, U.S.P. 4,243,802 and Japanese Patent Provisional Publication 55-11010 (corresponding to U.S. Application No. 11,613 of Feb. 2, 1990) propose the use of modified cellulose ethers whose viscosity is greatly increased by introducing long chain alkyl groups into a water-soluble cellulose ether having a relatively low degree of polymerization.

These modified water-soluble cellulose ethers are those obtained by modifying a water-soluble cellulose ether having a relatively low degree of polymerization with a modifier such as alkyl epoxides, alkyl halides or alkyl isocyanates having 10 to 24 carbon atoms. However, these modifiers are very expensive and thus cannot industrially be obtained easily. Moreover, they are liable to undergo hydrolysis and to easily corrode apparatuses used for the modification procedures. In addition, the handling thereof is very complicated and thus the use thereof is impractical.

Accordingly, an object of the present invention is to provide a modified water-soluble cellulose ether which is useful as a thickening agent for cement mortar, materials for extrusion molding, paints, cosmetic formulations, detergents or the like.

Another object of the present invention is to provide a method for preparing such a modified water-soluble cellulose ether.

The inventors of the present invention have conducted various studies under these circumstances, have found that a water-soluble cellulose ether showing very high viscosity-developing effect can be obtained if a glycidyl ether having a long chain alkyl group is employed as a modifier and thus have completed the present invention on the basis of such a finding.

According to an aspect of the present invention, there is provided a modified water-soluble cellulose ether which is obtained by reacting a water-soluble cellulose ether having a weight average molecular weight ranging from 10,000 to 3,000,000 and carrying at least one member selected from the group consisting of alkyl groups having 1 to 3 carbon atoms and hydroxyalkyl groups having 2 to 4 carbon atoms with a glycidyl ether carrying an alkyl group having 12 to 26 carbon atoms to thus substitute the hydroxyl groups in the cellulose ether molecule in a ratio ranging from 0.001 to 0.1 mole per mole of the glucose ring constituting the cellulose ether.

According to another aspect of the present invention, there is provided a method for preparing a modified water-soluble cellulose ether which comprises the step of reacting a water-soluble cellulose ether having a weight average molecular weight ranging from 10,000 to 3,000,000 and carrying at least one member selected from the group consisting of alkyl groups having 1 to 3 carbon atoms and hydroxyalkyl groups having 2 to 4 carbon atoms with a glycidyl ether carrying an alkyl group having 12 to 26 carbon atoms to thus substitute the hydroxyl groups in the cellulose ether molecule in a ratio ranging from 0.001 to 0.1 mole per mole of the glucose ring constituting the cellulose ether.

The modified cellulose ether obtained according to the present invention can effectively be used as a thickening agent for cement mortar, materials for extrusion molding, paints, cosmetic formulations, detergents or the like.

The water-soluble cellulose ethers can be arbitrarily selected from alkyl-, hydroxyalkyl- or hydroxyalkylalkyl celluloses having a weight average molecular weight ranging from 10,000 to 3,000,000 such as methyl cellulose, hydroxyethyl cellulose, hydroxyethyl methyl cellulose, hydroxypropyl methyl cellulose and hydroxyethyl ethyl cellulose. The number of the alkyl or hydroxyalkyl groups in these cellulose ethers is not restricted to a specific one so far as these cellulose ethers are water-soluble, but in particular the number of the alkyl groups in the cellulose ether is preferably, on the average, not more than 2.5 moles per mole of the glucose unit. This is because, if the number of the alkyl groups in the cellulose ether exceeds the limit, the modification of the cellulose ether becomes very difficult.

Moreover, examples of the glycidyl ethers carrying an alkyl group having 12 to 26 carbon atoms which may be used in the present invention include dodecyl glycidyl ether, hexadecyl glycidyl ether, octadecyl glycidyl ether, hexacosyl glycidyl ether.

According to the present invention, the modification of the cellulose ether may be performed in the same manner as that for the conventional method in which a conventionally known water-soluble cellulose ether is treated with a long chain alkyl epoxide. More specifically, a cellulose ether as a starting material is dispersed in an organic solvent such as a lower aliphatic alcohol, water and an alkali are added to the dispersion to swell the cellulose ether, then a glycidyl ether as a modifying agent is added to the mixture and the resulting mixture is stirred at a temperature ranging from 70 to 80 °c for 2 to 8 hours. The amount of the glycidyl ether to be used in the modification preferably ranges from 0.001 to 1.0 moles per mole of the glucose ring of the cellulose ether as the starting material. The reaction temperature and the reaction time are largely influenced by the intended degree of substitution with the modifying groups and they are appropriately selected depending on the degree of the substitution, while they fall within the foregoing ranges. After the reaction, the residual alkali is neutralized, the reaction product is washed with water and then dried.

In this respect, the degree of substitution with the modifying groups can be determined according to a modified Zeisel method. The modified Zeisel method comprises decomposing portions of the ether linkage of the resulting product with a solution of hydroiodic acid and the amount of the resulting alkyl iodide is determined by the gas chromatography technique.

The present invention will hereunder be explained in more specifically with reference to the following working Examples, but the present invention is not restricted to those specific Examples.

Example 1

There was dispersed, in 400 g of tertiary butyl alcohol, 40 g of hydroxypropyl methyl cellulose (METHOROSE 60SH-400, available from Shin-Etsu Chemical Co., Ltd.) whose degrees of substitution with hydroxypropoxyl groups and with methoxyl groups are 0.23 and 1.87 respectively, which has a weight average molecular weight of 180,000 and whose viscosity as determined at 20 °c on a 2 % aqueous solution thereof is 430 cps, 35 g of a 6 % aqueous solution of sodium hydroxide was added to the resulting dispersion and then the mixture was stirred for 2 hours in a nitrogen gas atmosphere. To the mixture, there was added 5 g of octadecyl glycidyl ether and the stirring was continued at 80 °c for additional 4 hours. After the reaction solution was neutralized with acetic acid, it was cooled and filtered to recover precipitated solid substances. The resulting solid substances were washed twice with 10 volume each of hexane and acetone and further with 100 volume of hot water to thus give a modified cellulose ether.

The degree of substitution with octadecyloxyhydroxypropyl groups (average number of substituents per glucose ring) thereof was 0.006. In addition, the viscosity of a 2 % aqueous solution of the resulting modified cellulose ether was determined using a Brookfield viscometer and was found to be 44,000 cps.

Example 2

There was dispersed, in 400 g of tertiary butyl alcohol, 40 g of methyl cellulose (METHOROSE SM-4,000, available from Shin-Etsu Chemical Co., Ltd.) whose degree of substitution with methoxyl groups is 1.84, which has a weight average molecular weight of 330,000 and whose viscosity as determined at 20 °c on a 2 % aqueous solution thereof is 4,100 cps, 35 g of a 6 % aqueous solution of sodium hydroxide was added to the resulting dispersion and then the mixture was stirred for 2 hours in a nitrogen gas atmosphere. To the mixture, there was added 20 g of dodecyl glycidyl ether and the stirring was continued at 80 °c for additional 10 hours. After the reaction solution was neutralized with acetic acid, it was cooled and filtered to recover solid materials. The resulting solid materials were washed twice with 10 volume each of hexane and acetone and further with 100 volume of hot water to thus give a modified cellulose ether.

The degree of substitution with dodecyloxyhydroxypropyl groups thereof was 0.060. The resulting modified cellulose ether was insoluble in water but was soluble in water-isopropanol (1:1 mixed solvent). In addition, the viscosity of a 2 % solution thereof in the solvent was found to be 12,000 cps at 20 °c.

Example 3

There was dispersed, in 400 g of tertiary butyl alcohol, 40 g of hydroxypropyl cellulose (SHINETSU HPC, available from Shin-Etsu Chemical Co., Ltd.) whose degree of substitution with hydroxypropoxyl groups is 2.80, which has a weight average molecular weight of 290,000 and whose viscosity as determined at 20 °c on a 2 % aqueous solution thereof is 240 cps, 35 g of a 6 % aqueous solution of sodium hydroxide was added to the resulting dispersion and then the mixture was stirred for 2 hours in a nitrogen gas atmosphere. To the mixture, there was added 20 g of $C_{24}$ to $C_{26}$ of alkyl glycidyl ether and the stirring was continued at 75 °c for additional 4 hours. After the reaction solution was neutralized with acetic acid, it was cooled, 800 g of hexane was added to the solution and the solution was filtered to recover precipitated solid substances. The resulting solid substances were washed twice with 10 volume each of hexane and acetone and further with 100 volume of hot water to thus give a modified cellulose ether. The degree of substitution with $C_{24}$ to $C_{26}$ of alkoxyhydroxypropyl groups thereof was 0.002. In addition, the viscosity (at 20 °c) of a 2 % aqueous solution of the resulting modified cellulose ether was found to be 9,300 cps.

Example 4

There was dispersed, in 400 g of tertiary butyl alcohol, 40 g of hydroxypropyl cellulose (METHOROSE 90SH-100,000, available from Shin-Etsu Chemical Co., Ltd.) whose degrees of substitution with hydroxypropoxyl groups and with methoxyl groups are 0.18 and 1.44 respectively, whose viscosity as determined at 20 °c on a 2 % aqueous solution thereof is 67,000 cps, 70 g of a 6 % aqueous solution of sodium hydroxide was added to the resulting dispersion and then the mixture was stirred for 2 hours in a nitrogen gas atmosphere. To the mixture, there was added 5 g of octadecyl glycidyl ether and the stirring was continued at 80 °c for additional 6 hours. After the reaction solution was neutralized with acetic acid, it was cooled and filtered to recover precipitated solid substances. The resulting solid substances were washed twice with 10 volume each of hexane and acetone and further with 100 volume of hot water to thus give a modified cellulose ether. The degree of substitution with octadecyloxyhydroxypropyl groups thereof was 0.009. The resulting modified cellulose ether was insoluble in water but was soluble in water-isopropanol (4:1 mixed solvent) and the viscosity of a 2 % aqueous solution was determined using a Brookfield viscometer at 20 °c and was found to be 230,000 cps.

A modified water-soluble cellulose ether shows a high viscosity-developing effect in only a small amount and is useful as a thickening agent for cement mortar, materials for extrusion molding, paints, cosmetic formulations, detergents or the like. The modified cellulose ether can be obtained according to a method comprising the step of reacting a water-soluble cellulose ether having a weight average molecular weight ranging from 10,000 to 3,000,000 and carrying alkyl groups having 1 to 3 carbon atoms and/or hydroxyalkyl groups having 2 to 4 carbon atoms with a glycidyl ether carrying an alkyl group having 12 to 26 carbon atoms to thus substitute the hydroxyl groups in the cellulose ether molecule in a ratio ranging from 0.001 to 0.1 mole per mole of the glucose ring constituting the water-soluble cellulose ether.

**Claims**

1. A modified water-soluble cellulose ether which is obtained by reacting a water-soluble cellulose ether having a weight average molecular weight ranging from 10,000 to 3,000,000 and carrying at least one member selected from the group consisting of alkyl groups having 1 to 3 carbon atoms and hydroxyalkyl groups having 2 to 4 carbon atoms with a glycidyl ether carrying an alkyl group having 12 to 26 carbon atoms to thus substitute the hydroxyl groups in the cellulose ether molecule in a ratio ranging from 0.001 to 0.1 mole per mole of the glucose ring constituting the water-soluble cellulose ether.

2. The modified water-soluble cellulose ether as claimed in claim 1 wherein the water-soluble cellulose ether is a member selected from the group consisting of methyl cellulose, hydroxyethyl cellulose, hydroxyethyl methyl cellulose, hydroxypropyl methyl cellulose and hydroxyethyl ethyl cellulose.

3. The modified water-soluble cellulose ether as claimed in claim 1 wherein the amount of the alkyl groups of the water-soluble cellulose ether is, on the average, not more than 2.5 moles per mole of the glucose unit.

4. The modified water-soluble cellulose ether as claimed in claim 1 wherein the glycidyl ether carrying an alkyl group having 12 to 26 carbon atoms is a member selected from the group consisting of dodecyl glycidyl ether, hexadecyl glycidyl ether, octadecyl glycidyl ether, hexacosyl glycidyl ether.

5. A method for preparing a modified water-soluble cellulose ether comprising the step of reacting a water-soluble cellulose ether having a weight average molecular weight ranging from 10,000 to 3,000,000 and carrying at least one group selected from the group consisting of alkyl groups having 1 to 3 carbon atoms and hydroxyalkyl groups having 2 to 4 carbon atoms with a glycidyl ether carrying an alkyl group having 12 to 26 carbon atoms to thus substitute the hydroxyl groups in the cellulose ether molecule in a ratio ranging from 0.001 to 0.1 mole per mole of the glucose ring constituting the water-soluble cellulose ether.

6.  The method for preparing a modified water-soluble cellulose ether as claimed in claim 5 wherein the water-soluble cellulose ether is a member selected from the group consisting of methyl cellulose, hydroxyethyl cellulose, hydroxyethyl methyl cellulose, hydroxypropyl methyl cellulose and hydroxyethyl ethyl cellulose.

7.  The method for preparing a modified water-soluble cellulose ether as claimed in claim 5 wherein the amount of the alkyl groups of the water-soluble cellulose ether is, on the average, not more than 2.5 moles per mole of the glucose unit.

8.  The modified water-soluble cellulose ether as claimed in claim 5 wherein the glycidyl ether carrying an alkyl group having 12 to 26 carbon atoms is a member selected from the group consisting of dodecyl glycidyl ether, hexadecyl glycidyl ether, octadecyl glycidyl ether, hexacosyl glycidyl ether.

9.  The method for preparing a modified water-soluble cellulose ether as claimed in claim 5 wherein the amount of the glycidyl ether, which is required for substituting the hydroxyl groups in the cellulose ether molecule in a ratio ranging from 0.001 to 0.1 mole per mole of the glucose ring constituting the water-soluble cellulose ether, ranges from 0.01 to 1.0 moles per mole of glucose ring of the cellulose ether.

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| E | EP-A-0 390 240 (BEROL NOBEL AB)<br>* Page 4, lines 52-55; page 6, line 21 - page 7, line 35; page 10; example F; claims 6,7 *<br>– – – | 1-8 | C 08 B 11/193 |
| X | EP-A-0 295 628 (AQUALON)<br>* Page 2, lines 41-45; page 3, lines 4-19; claims *<br>– – – | 5,6 | |
| A | JOURNAL OF POLYMER SCIENCE: PART C, no. 36, 1971, pages 491-508, John Wiley<br>& Sons, Inc.; E.D. KLUG: "Some properties of water-soluble hydroxyalkyl celluloses and their derivatives"<br>* Page 497, line 19 - page 498, line 4 *<br>– – – | 1,2,5,6 | |
| A | US-A-4 228 277 (L.M. LANDOLL)<br>– – – – – | | |

| TECHNICAL FIELDS SEARCHED (Int. Cl.5) |
|---|
| C 08 B |

| Place of search | Date of completion of search | Examiner |
|---|---|---|
| The Hague | 16 April 91 | MAZET J.-F. |

The present search report has been drawn up for all claims